# EUROPÄISCHE PATENTSCHRIFT

(11) **EP 2 734 287 B1**
(45) Veröffentlichungstag und Bekanntmachung des Hinweises auf die Patenterteilung: **27.03.2019**
(21) Anmeldenummer: 12738040.0
(22) Anmeldetag: 17.07.2012
(51) Int. Cl.: B01D 61/58, B01D 63/04

(54) **FILTERMODUL**
FILTER MODULE
MODULE DE FILTRAGE

(30) Priorität: 18.07.2011 DE 102011107980; 18.07.2011 US 201161457957 P
(43) Veröffentlichungstag der Anmeldung: 28.05.2014
(73) Patentinhaber: Fresenius Medical Care Deutschland GmbH, 61352 Bad Homburg (DE)
(72) Erfinder: KELLER, Torsten, 54411 Hermeskeil (DE); KUGELMANN, Franz, 66606 Sankt Wendel (DE); WEHMEYER, Wolfgang, 72076 Tübingen (DE)
(74) Vertreter: Laufhütte, Dieter
(86) Internationale Anmeldenummer: PCT/EP2012/003012
(87) Internationale Veröffentlichungsnummer: WO 2013/010666

(56) Entgegenhaltungen:
- EP-A1- 0 765 683
- EP-A1- 1 466 657
- EP-A2- 0 076 421
- EP-A2- 0 791 368
- WO-A1-02/47785
- DE-A1- 3 326 704
- DE-A1- 3 901 446

## Beschreibung

Die vorliegende Erfindung betrifft ein Filtermodul sowie ein Verfahren zur Herstellung eines Hohlfasermembranbündels mit wenigstens zwei konzentrisch angeordneten Filterstufen. Insbesondere betrifft die Erfindung Filtermodule für die Hämodialyse, insbesondere Filtermodule, die mehrere Filtrationsstufen integral enthalten.

Für viele therapeutische Verfahren der extrakorporalen Blutreinigung sind mehrere Filter notwendig. Das Blut wird in diesen Verfahren zur Reinigung von toxischen Metaboliten in der Regel an Hohlfasermembranen eines Filtermoduls filtriert oder dialysiert. Den dabei zur Substitution oder zur Dialyse eingesetzten Flüssigkeiten sind hohe Anforderungen gestellt, um in den therapeutischen Verfahren eingesetzt werden zu können. Solche Flüssigkeiten werden in Beuteln mit arzneimittelrechtlicher Zulassung bereitgestellt oder von den extrakorporalen Blutbehandlungsmaschinen online hergestellt. Um bei den Online-Verfahren die mikrobiologische Reinheit gewährleisten zu können, müssen diese Flüssigkeiten eine oder zwei Filtrationsstufen durchlaufen, ehe sie als Austauschflüssigkeit in der Dialyse oder als Substitutionsflüssigkeit in Blutfiltrationsverfahren eingesetzt werden können.

Bislang gibt es die Bestrebungen, die Flüssigkeitskreisläufe der extrakorporalen Blutbehandlungskreisläufe kompakter zu gestalten und auf Einwegartikeln zu integrieren. Insbesondere sind Kassettensysteme für die extrakorporale Blutbehandlung entwickelt worden, auf denen eine Vielzahl von Funktionen zur Blut- und Flüssigkeitsbehandlung integriert sind.

Ebenso sind auf dem Gebiet der Blutfilter mit Hohlfasermembranen bislang Entwicklungen vorangetrieben worden, bei denen mehrere Filterstufen in einem Filtermodul integriert wurden.

Die EP 0 791 368 A2 zeigt ein zweistufiges Filtermodul für die Hämodiafiltration. In einer ersten Stufe dringt einströmende Substitutionsflüssigkeit in das Innere der Hohlfasermembranen ein, wird dabei filtriert und in einer Blutkammer am Kopfende des Filtermoduls mit Blut vermischt. In einer zweiten Stufe durchläuft das verdünnte Blut das Innere der Hohlfasermembranen und wird dabei dialysiert und filtriert.

Die DE 39 01 446 A1 beschreibt eine Hemodiafiltrationspatrone, mit einer ersten Einrichtung mit semipermeabler Membran, um das Dialysat vor seinem Kontakt mit Blut zu sterilisieren und depyrogenisieren und einer zweiten Einrichtung mit semipermeabler Membran zur Entfernung von Abfallstoffen vom Blut, wobei die zweite Einrichtung mitsemipermeabler Membran eine Vielzahl von semipermeablen Hohlfasern enthält, wobei alle diese Hohlfasern an beiden Enden offen sind und diese Vielzahl von semipermeablen Hohlfasern sich generell in Längsrichtung durch die Patrone vom ersten Ende der Patrone zum zweiten Ende derPatrone erstreckt, wobei die semipermeablen Hohlfasern zwei Enden haben, von denen ein Ende in Verbindung mit dem Bluteinlaß und das andere Ende in Verbindung mit dem Blutauslaß steht und die erste Einrichtung mitsemipermeabler Membran eine Vielzahl von semipermeablenHohlfasern enthält, wobei alle diese Hohlfasern am gleichen Ende offen sind und am entgegengesetzten Ende geschlossen sind und wobei sich die Vielzahl von semipermeablen Hohlfasern generell in Längsrichtung in der Patrone vom ersten Ende der Patrone zum zweiten Ende der Patrone erstreckt, und sich die offenen Enden der Hohlfasern der ersten Einrichtung mit semipermeabler Membran zum ersten Ende der Patrone erstrecken und in Verbindung mit dem Dialysateinlaß stehen, damit Dialysat in die Hohlfasernhineinfließen kann. Dabei ist vorgesehen, dass die Dialysatkammer und die Blutkammer im Innern der Patrone durch eine erste Sperreinrichtung teilweise voneinander getrennt sind, die sich imwesentlichen in Längsrichtung in der Patrone von den geschlossenen Enden der Hohlfasern der ersten Einrichtung mit semipermeabler Membran zum ersten Ende der Patrone erstreckt, aber das erste Ende der Patrone nicht erreicht, wobei der Zwischenraum zwischen dem Ende der ersten Sperreinrichtung und dem ersten Ende der Patrone dazu dient, einen Dialysatfluß von der Dialysatkammer indie Blutkammer zu ermöglichen.

Die DE 33 26 704 A1 zeigt ein zweistufiges Filtermodul, in dem die Hohlfasermembranbündel konzentrisch angeordnet sind. In einer inneren ersten Filtrationsstufe wird Blut filtriert. In der zweiten umgebenden Filtrationsstufe wird Blut im Stoffaustausch mit Dialyseflüssigkeit behandelt.

Weitere Filter sind aus der EP 0 076 421 A2, der EP 0 765 683 A, der EP 1 466 657 A1 und der WO 02/47785 A1 bekannt.

Es ist daher die Aufgabe der vorliegenden Erfindung, ein Filtermodul sowie ein Verfahren zur Herstellung eines Hohlfasermembranbündels mit zwei konzentrisch angeordneten Filterstufen in vorteilhafter Weise weiterzubilden, insbesondere dahingehend, dass der Aufbau eines medizinischen Flüssigkeitskreislaufs unter Einsatz des Filtermoduls vereinfacht werden kann.

Diese Aufgabe wird erfindungsgemäß gelöst durch ein Filtermodul mit den Merkmalen des Anspruchs 1. Danach ist vorgesehen, dass ein Filtermodul mit wenigstens einer ersten und wenigstens einer zweiten Filterstufe bereitgestellt wird, umfassend und/oder bestehend aus Hohlfasermembranen, die im Wesentlichen konzentrisch angeordnet sind, wobei die Faserenden der Hohlfasermembranen zumindest teilweise und/oder zumindest einseitig in einer Vergussmasse eingebettet sind, wobei die erste Filterstufe eine Filterstufe zur Filtration einer ersten medizinischen Flüssigkeit und die zweite Filterstufe eine Filterstufe zur Filtration einer zweiten medizinischen Flüssigkeit ist, wobei die erste Filterstufe einseitig verschlossene Faserenden aufweist, wobei ein die erste Filterstufe umgebendes Begrenzungsmittel vorgesehen ist, das die erste Filterstufe und die zweite Filterstufe zumindest teilweise voneinander trennt und wenigstens eine Öffnung aufweist, die eine Fluidverbindung zwischen erster Filterstufe und zweiter Filterstufe herstellt. Dabei ist die Öffnung im Begrenzungsmittel aus einer oder einer Mehrzahl von umfänglich an dem Begrenzungsmittel angeordneten Öffnungen gebildet.
Dadurch ergibt sich der Vorteil, dass eine integrierte Anordnung von einem Ultrafilter und einem Blutfilter in einem einzigen Filtergehäuse eines Filtermoduls erreicht werden kann. Hierdurch ergibt sich insbesondere der Vorteil, dass eine Kostenreduktion durch geringeren Materialeinsatz erreicht werden kann, da nunmehr für einen einzigen Filter zwei bis drei Filter integriert werden können bzw. die Funktion von zwei bis drei Filtern durch ein einziges Filtermodul erreicht werden kann.

Darüber hinaus ist es möglich, ein extrudiertes Gehäuse zu verwenden, wodurch im Vergleich zu Spritzgussherstellverfahren eine Kostenreduktion erreicht werden kann. Des Weiteren ist es möglich, durch einen vereinfachten Aufbau, der sich durch das erfindungsgemäße Filtermodul ergibt, insgesamt den Aufbau einer Blutbehandlungsvorrichtung wie einer Dialysemaschine zu vereinfachen, etwa dahingehend, dass weniger Ventile, Schläuche, Elektronik oder Software benötigt bzw. in weniger komplexer Form benötigt wird bzw. werden. Weiter ergibt sich ein Vorteil dadurch, dass das Filtermodul vollständig als Einwegartikel, also als ein sogenanntes Disposable ausgeführt werden kann, wodurch insgesamt eine höhere Sicherheit durch Produkteinzeltests in der Produktion vor jeder Verwendung bei geringeren Kosten erreicht werden kann. Insbesondere ist es nicht mehr nötig, eine Wiederverwendung von Sterilfiltern auf der Dialysatseite einer Blutbehandlungsvorrichtung wie einer Dialysemaschine vorzunehmen.

Ferner ist es möglich, eine Reduktion von Testzeiten während der Dialyse zu erreichen, wofür bislang Filterintegritätstests herangezogen werden müssen, da die Sterilfilter auf der Dialysatseite wiederverwendet werden. Hierdurch kann eine höhere Dialysedosis für den Patienten erreicht werden. Zugleich ist es möglich, eine höhere Dialysedosis bei gleicher blutdurchströmter Fläche zu erreichen, da mittels des erfindungsgemäßen Filtermoduls eine bessere Dialysatverteilung erreicht werden kann.

Es kann vorgesehen sein, dass die erste medizinische Flüssigkeit eine Dialysier- und/oder Substitutionsflüssigkeit ist und/oder dass die zweite medizinische Flüssigkeit Blut ist und/oder dass die erste Filterstufe von der zweiten Filterstufe umgeben ist. Dabei kann vorgesehen sein, dass die erste Filterstufe in Form eines im Wesentlichen zylindrischen Hohlfaserbündels ausgeführt ist, während die zweite Filterstufe in Form eines hohlzylindrischen Hohlfaserbündels ausgeführt ist, in dem die erste Filterstufe angeordnet ist.

Insbesondere ist vorgesehen, dass die zweite Filterstufe jeweils auf beiden Seiten unverschlossene Faserenden aufweist.

Darüber hinaus ist möglich, dass das Begrenzungsmittel eine Begrenzungswand aufweist und/oder ausbildet und dass die Öffnung im Begrenzungsmittel in der Begrenzungswand im zur Vergussmasse benachbarten Bereich angeordnet ist und/oder dass das Begrenzungsmittel wenigstens eine Entlüftungsöffnung aufweist. Öffnungen in der Begrenzungswand im zur Vergussmasse benachbarten Bereich können umfänglich in der Begrenzungswand angeordnet sein, z. B. durch Perforierung und/oder Durchlochung dieses Abschnittes der Begrenzungswand. Damit erfolgt umfänglich der ersten Filterstufe ein gleichmäßiger Übergang des Filtrats in die zweite Filterstufe und eine optimierte Anströmung der Fasern in der zweiten Filterstufe. Eine Entlüftungsöffnung, die vorzugsweise mit einem vergleichsweise zur im Begrenzungsmittel angeordneten Öffnung kleineren Öffnung ausgeführt ist, ist insbesondere dann sinnvoll, wenn das Filtermodul leicht schräg gestellt wird, wobei die Bluteintrittsseite etwas höher steht. Hierdurch wird die Entlüftung des Filtermoduls erleichtert. Da jedoch während des Betriebs hier eine kleine Menge Dialysat direkt zum Dialysatausgang des Filtermoduls fließen kann, wodurch die Leistung negativ beeinflusst werden könnte, ist es zweckmäßig, eine vergleichsweise kleine Öffnung als Entlüftungsöffnung vorzusehen. Grundsätzlich ist auch denkbar, dass mehrere Öffnungen im Begrenzungsmittel vorgesehen sind, wobei die Öffnungen umlaufend um den Umfang des Begrenzungsmittels angeordnet sind. Hierdurch wird es möglich, eine besonders gleichmäßige Verteilung des Dialysats zu erreichen, so dass die zweite Filterstufe auf ihrer Außenseite gleichmäßig von Dialysat umströmt wird.

Es ist möglich, dass das Begrenzungsmittel eine Folie und/oder ein Rohr ist oder umfasst.

In einer bevorzugten Ausfürhungsform handelt es sich bei den Öffnungen um umfänglich angeordnete Schlitze, Perforierungen, Löcher, Labyrinthschlitze oder dergleichen.

Des Weiteren kann vorgesehen sein, dass das Filtermodul eine erste Endkappe aufweist, in der ein Anschluss zur ersten Filterstufe und ein Anschluss zur zweiten Filterstufe angeordnet ist, wobei die Faserenden der ersten Filterstufe auf der Seite der ersten Endkappe unverschlossen sind und wobei wenigstens ein Dichtelement vorgesehen ist, mittels dessen die unverschlossenen Faserenden der ersten Filterstufe vom Anschluss zur zweiten Filterstufe und von der zweiten Filterstufe abdichtbar und/oder abgedichtet sind.

Ferner ist möglich, dass das Filtermodul eine zweite Endkappe aufweist, in der ein Anschluss zur zweiten Filterstufe angeordnet ist und/oder dass die Faserenden der ersten Filterstufe auf der Seite der zweiten Endkappe verschlossen sind.

Außerdem kann vorgesehen sein, dass das Filtermodul ein Gehäuse aufweist, in dem die Filterstufen angeordnet sind, wobei im Gehäuse wenigstens ein Anschluss zur ersten Filterstufe angeordnet ist.

Grundsätzlich kann es sich bei einem Anschluss um einen Zulauf oder einen Ablauf zu einer Filterstufe handeln, da es grundsätzlich möglich ist, das Filtermodul sowohl nach der einen als auch nach der anderen Fluidrichtung zu betreiben, also die Filterstufen sowohl in der einen als auch in der anderen Richtung mit einem Fluid zu durchströmen.

Darüber hinaus kann vorgesehen sein, dass konzentrisch und koaxial zur Längsachse des Filtermoduls und/oder zu den Filterstufen wenigstens ein Kanalelement vorgesehen ist, das mit der zweiten Filterstufe verbunden ist und/oder dass auf der Außenseite des Filtermoduls wenigstens ein Kanalelement vorgesehen ist, das mit der zweiten Filterstufe verbunden ist.

Weiter ist denkbar, dass das Filtermodul wenigstens ein Pumpenelement aufweist und/oder mit wenigstens einem Pumpenelement verbindbar ist, mittels dessen die erste und/oder zweite medizinische Flüssigkeit pumpbar ist.

Außerdem kann vorgesehen sein, dass das Pumpenelement ein in das Filtermodul integriertes Pumpenelement ist und/oder dass das Pumpenelement eine Impellerpumpe ist.

Des Weiteren ist möglich, dass eine dritte Filterstufe vorgesehen ist, wobei die erste und die dritte Filterstufe derart zueinander angeordnet sind, dass die erste medizinische Flüssigkeit aus der ersten Filterstufe über und/oder durch die Hohlfasermembranen der ersten und dritten Filterstufe in die dritte Filterstufe einleitbar ist.

Weiter ist denkbar, dass die erste, zweite und dritte Filterstufe konzentrisch und koaxial angeordnet sind, wobei die erste Filterstufe die dritte Filterstufe umgibt und die zweite Filterstufe die erste Filterstufe umgibt.

Außerdem kann vorgesehen sein, dass das Filtermodul eine erste Endkappe aufweist, in der ein Anschluss zur ersten Filterstufe und ein Anschluss zur zweiten Filterstufe angeordnet ist, wobei die Faserenden der ersten Filterstufe auf der Seite der ersten Endkappe unverschlossen sind und wobei wenigstens ein Dichtelement vorgesehen ist, mittels dessen die unverschlossenen Faserenden der ersten Filterstufe vom Zulauf zur ersten Filterstufe und von der zweiten Filterstufe abdichtbar und/oder abgedichtet sind, wobei die Faserenden der dritten Filterstufe auf der Seite der ersten Endkappe verschlossen sind, wobei eine zweite Endkappe vorgesehen ist, in der ein Anschluss der dritten Filterstufe angeordnet ist und wobei die Faserenden der dritten Filterstufe auf der Seite der zweiten Endkappe unverschlossen sind.

Ferner kann vorgesehen sein, dass an den Anschluss zur dritten Filterstufe wenigstens ein Pumpenelement anschließbar und/oder angeschlossen ist, wobei das Pumpenelement vorzugsweise ein integriertes Pumpenelement ist, und/oder wobei der Anschluss zur dritten Filterstufe mit einem Anschluss zur zweiten Filterstufe verbindbar und/oder verbunden ist.

Des Weiteren betrifft die vorliegende Erfindung ein Verfahren zur Herstellung eines Hohlfasermembranbündels mit zwei konzentrisch angeordneten Filterstufen mit den Merkmalen des Anspruchs 16. Danach ist vorgesehen, dass bei einem Verfahren zur Herstellung eines Hohlfasermembranbündels mit wenigstens zwei konzentrisch angeordneten Filterstufen wenigstens die folgenden Schritte umfasst sind:
- Einfassen eines ersten Hohlfasermembranbündels für eine erste Filterstufe in einer Folie, Schlauchfolie und/oder einem Rohr;
- Einbetten der ersten Filtrationsstufe in Hohlfasern der zweiten Filtrationsstufe;
- Vergießen der ersten und der zweiten Filtrationsstufe durch eine Vergussmasse in einem Gießprozess;
- Abschneiden eines Endbereichs der Vergussmasse inklusive verschlossener Faserenden zum Freilegen und Offenlegen aller Faserenden; und
- Verschließen einer Faserendenseite der ersten Filtrationsstufe durch Wärmeeinwirkung und Schmelzen der Faserendenseite und Vergussmasse.

Prozesse zum Vergießen von Hohlfaserbündeln sind im Stand der Technik bekannt, etwa z. B. aus der JP 06-006156. Zunächst müssen die Faserenden verschlossen werden, so dass keine Vergussmasse in die Faser eindringen kann. Dies passiert bereits durch Abschmelzverfahren mit Hitzeinwirkung auf die Faserenden, z. B. durch Laserbestrahlung, Hitzespiegel oder Heißstempel. Es erfolgt anschließend der Verguss mit einer aushärtenden vergießbaren Masse, z. B. Polyurethanmaterialien, so dass die Faserenden bis zu einigen Zentimetern durch die Vergussmasse umschlossen sind. Es erfolgt anschließend ein Schnitt durch die Vergussmasse am äußeren Ende der Faserenden, so dass die in der Vergussmasse eingebetteten verschlossenen Faserenden abgeschnitten werden. Übrig bleibt das am Ende vergossene Faserbündel mit nun offenen Faserenden.

Um wie in der vorliegenden Erfindung die Enden einer Filtrationsstufe in dem Herstellungsverfahren wieder zu verschließen, muss ein weiterer Schritt des Verschließens der Hohlfaserenden folgen. Durch erneute geometrisch präzise Einwirkung von Hitze auf eine Zone des mehrere Filtrationsstufen umfassenden Hohlfaserbündels kann eine Filtrationsstufe durch Schmelzen der Vergussmasse mit Faserenden verschlossen werden. Die Hitzeeinwirkung kann beispielsweise durch Laser, Hitzespiegel oder Hitzestempel oder dergleichen erfolgen.

Insbesondere kann vorgesehen sein, dass das Verschließen der Faserendenseite mittels Laser und/oder Heißstempel erfolgt.

Denkbar ist ferner, dass in der ersten Filtrationsstufe eine dritte Filtrationsstufe ausgebildet wird, wobei auf einer Faserendenseite ein erster Bereich verschlossen wird und wobei auf der anderen Faserendenseite ein zweiter Bereich verschlossen wird, wobei im ersten Bereich Hohlfasern verschlossen werden und diese Hohlfasern auf der anderen Faserendenseite vom zweiten Bereich umgeben und zumindest teilweise unverschlossen sind.

Hierdurch kann erreicht werden, dass zunächst auf der einen Faserendenseite das Fluid, z. B. das Dialysat in die erste Filterstufe eintritt und aufgrund der verschlossenen Faserenden nicht in die dritte Filterstufe eintreten kann. Ein Übertritt des Fluids von der ersten in die dritte Filterstufe kann somit vorteilhafterweise nur über die Membranen der Hohlfasermembranen der ersten und dritten Filterstufe erfolgen. Da auf der anderen Faserendenseite die Hohlfaserenden der ersten Filterstufe verschlossen und die Hohlfaserenden der dritten Filterstufe unverschlossen sind, kann das Fluid nicht ungefiltert übertreten, sondern muss durch die Membranen strömen. Hierdurch wird das Fluid, z. B. eine medizinische Flüssigkeit wie Dialysat, sicher gefiltert.

Es ist möglich, dass in der ersten Filtrationsstufe eine dritte Filtrationsstufe ausgebildet wird, wobei auf einer Faserendenseite ein erster Bereich verschlossen wird und wobei auf der anderen Faserendenseite ein zweiter Bereich verschlossen wird, wobei im ersten Bereich Hohlfasern verschlossen werden und diese Hohlfasern auf der anderen Faserendenseite vom zweiten Bereich umgeben und zumindest teilweise unverschlossen sind und dass der erste Bereich kreisförmig und dass der zweite Bereich kreisringförmig ist, wobei vorzugsweise der Innendurchmesser des zweiten Bereich kleiner gewählt ist als der Außendurchmesser des ersten Bereichs. Der Austrittsdurchmesser des Faserbündels der dritten Filterstufe wird hierdurch beispielsweise kleiner ausgeführt als der Kerndurchmesser des vergossenen bzw. verschlossenen Bündels auf der Eintrittsseite der dritten Filterstufe. Damit wird vorteilhaft gewährleistet, dass Dialysat immer in zwei Stufen gefiltert wird, bevor es z. B. als Post- und/oder Predilution in den extrakorporalen Blutkreislauf infundiert wird. Insbesondere kann dadurch erreicht werden, dass einzelne Fasern, die durch die Produktion unversehens schräg in dem Gesamtbündel liegen, nicht von der dritten Filterstufe in die erste Filterstufe hereinragen. Dadurch lässt sich einfach, zuverlässig und vorteilhaft eine zweistufige Filtration gewährleisten.

Darüber hinaus kann vorgesehen sein, dass in wenigstens einem weiteren Schritt aus dem Hohlfasermembranbündel ein Filtermodul nach einem der Ansprüche 1 bis 15 hergestellt wird.

Ferner betrifft die vorliegende Erfindung ein Disposable mit den Merkmalen des Anspruchs 21. Danach ist vorgesehen, dass ein Disposable wenigstens ein Filtermodul nach einem der Ansprüche 1 bis 15 umfasst.

Des Weiteren kann vorgesehen sein, dass das Disposable eine disposable Kassette ist und/oder umfasst.

Ferner betrifft die vorliegende Erfindung eine Blutbehandlungsvorrichtung mit den Merkmalen des Anspruchs 22. Danach ist vorgesehen, dass eine Blutbehandlungsvorrichtung wenigstens ein Filtermodul nach einem der Ansprüche 1 bis 15 und/oder ein Disposable nach Anspruch 21 aufweist.

Weitere Einzelheiten und Vorteile der Erfindung sollen nun anhand eines in der Zeichnung dargestellten Ausführungsbeispiels näher erläutert werden.

Es zeigen
- Fig.1:: eine schematische Darstellung eines Filtermoduls durch einen Schnitt entlang der zentralen Symmetrieachse der konzentrischen Faserbündel;
- Fig. 2:: eine schematische Draufsicht auf die Bluteintrittseite;
- Fig. 3:: eine schematische Draufsicht auf die Blutaustrittseite;
- Fig. 4:: eine weitere schematische Darstellung eines Filtermoduls in einer zweiten Ausführungsform durch einen Schnitt entlang der zentralen Symmetrieachse der konzentrischen Faserbündel;
- Fig. 5:: eine weitere schematische Darstellung eines Filtermoduls in einer dritten Ausführungsform durch einen Schnitt entlang der zentralen Symmetrieachse der konzentrischen Faserbündel; und
- Fig. 6:: eine schematische Darstellung eines Filtermoduls in einer vierten Ausführungsform durch einen Schnitt entlang der zentralen Symmetrieachse der konzentrischen Faserbündel;
- Fig. 7:: eine schematische Draufsicht auf die Bluteintrittseite; und
- Fig. 8:: eine schematische Draufsicht auf die Blutaustrittseite.

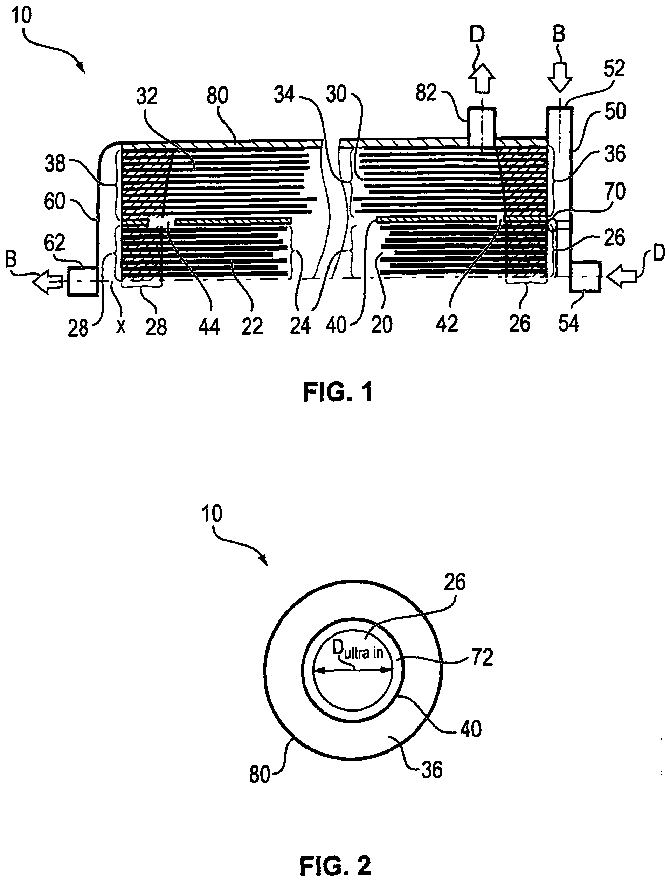

Figur 1 zeigt eine schematische Darstellung eines einstufigen, integrierten Ultrafilters als erster Filterstufe 20 in einem Filtermodul 10, wobei nur die obere Hälfte des zur Achse X im Wesentlichen radialsymmetrischen Filtermoduls 10 dargestellt ist.

Der Kern des Faserbündels der ersten Filterstufe 20 besteht aus den Ultrafiltermembranen 22. Diese können identisch zu den Blutfiltermembranen 32 der zweiten Filterstufe 30 sein, jedoch auch in Membranstruktur (hoher KUF für Wasser; KUF = Ultrafiltrationskoeffizient) und Dimensionen (kleiner Innendurchmesser) an die Aufgabe der Wasserfiltration angepasst sein.

Dieses Faserbündel 24 der ersten Filterstufe 20 ist von einem Begrenzungsmittel 40 in Form einer Schlauchfolie oder einem Rohr umgeben, das ein Gehäuse mit einseitigem Auslass 44 darstellt. Die Öffnung 44 kann mehrere Öffnungen umfänglich am Rohr 40 umfassen, so dass ein radial gleichmäßiger Übertritt des Filtrats aus der ersten Filterstufe 20 in die zweite Filterstufe 30 erfolgt. Das Dialysat tritt stirnseitig durch den in der Endkappe 50 angeordneten Anschluss 54, der ein Dialysateinlass 54 ist, durch die unverschlossenen Hohlfasermembranenden 26 in das Lumen der ersten Filterstufe 20 ein, die eine Ultrafilterstufe ist und tritt durch die Wandung in den inneren Bereich des Faserbündels 24 aus, da die Enden 28 der Hohlfasern 22 ersten Filterstufe 20 auf der Seite der zweiten Endkappe 60 vollständig verschlossen sind.

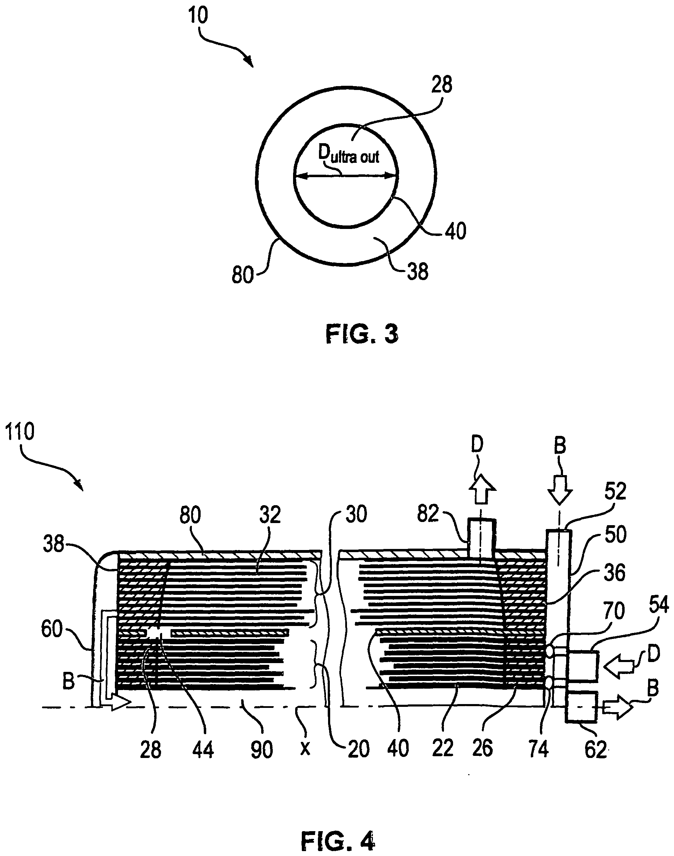

Das Faserbündel 24 wird vor dem Verschluss z. B. mit einem Laser oder Heissstempel auf beiden Stirnseiten so versiegelt, dass die Ultrafiltermembranen 22 der ersten Filterstufe nach dem Verschließen einseitig verschlossene Fasern erhalten, nämlich im Endbereich 28, während der Endbereich 26 unverschlossen ist. Um ein direktes Überströmen von Dialysat durch einzelne Fasern in den Dialysat- oder Blutraum sicher auszuschließen, ist der Eintrittsdurchmesser für das Dialysat durch einen Dichtring 70 etwas kleiner, als der Austrittsdurchmesser (vgl. Figuren 2 und 3). Der Dichtring 70 liegt auf den Ultrafilterfasern auf der Auflagefläche 72 auf und verschließt sie.

Das Fasermodul 10 wird auf der Eintrittsseite mit der speziell ausgeführten Endkappe 50 versehen, in der zentral über den Anschluss 54 das Dialysat und radial über den Anschluss 52 das Blut zugeführt wird. Der Dialysataustritt 82 befindet sich hier radial am Gehäuse 80. Die Pfeile bezeichnen die Strömungsrichtung D des Dialysats und die Strömungsrichtung des Blutes B.

Auf der Blutaustrittsseite wird das Modul mit einer Standardkappe 60 mit zentralem Blutaustritt 62 versehen. Grundsätzlich ist auch denkbar, dass der Blutaustritt radial angeordnet ist.

Da das Dialysat im Kern des Faserbündels 24 austritt, ist eine gute Dialysatverteilung ohne Durchmesservergrößerung des Gehäuses an den Enden möglich. Es ist also ein einfaches Rohr als Gehäuse denkbar, welches entweder in eine Kassette integriert ist, oder kostengünstig extrudiert wurde.

Das Filtermodul 10 sollte horizontal liegend betrieben werden, um eine gute Entlüftung beim Primen zu gewährleisten. Der Dialysatausgang 82 und der (radiale) Blutausgang sollten nach oben weisen.

Hilfsweise kann das Modul leicht schräg gestellt werden, wobei die Bluteintrittseite etwas höher steht. In diesem Fall ist es sinnvoll, das innere Rohr bzw. Begrenzungsmittel 40, welches den Ultrafilter 20, also die erste Filterstufe, vom Blutfilter 30, also der zweiten Filterstufe 30 trennt, an der Eintrittsseite des Dialysats mit einer kleinen Entlüftungsöffnung 42 zu versehen. Während des Betriebs wird hier eine kleine Menge Dialysat direkt zum Ausgang fließen und die Leistung negativ beeinflussen. Die Öffnung sollte daher vorzugsweise sehr klein gehalten werden.

Die Hohlfasermembranen 32 der zweiten Filterstufe 30 bilden ein im Wesentlichen hohlzylindrisches Faserbündel 34, in dem das Bündel 24 angeordnet ist. Beide Enden 36, 38 des Faserbündels 34 sind unverschlossen.

Die Fasern 32 für die Blutreinigung können eine Ausrichtung haben, die nicht parallel zur Achse X des Filtermoduls 10 ist. Die Fasern 32 laufen dann am Umfang mit einem Winkel zur Längsachse um das Kernmodul (Ultrafilter) und haben so eine größere effektive Länge. Hierdurch würde sich die optimale Konstruktion eines Ultrafilters (viele kurze Fasern) mit der eines auf Diffusion angewiesenen Blutfilters (längeres Bündel) kombinieren lassen. Diese Schrägverlegung der Blutfilterfasern kann in gegenläufiger Richtung verlaufen, ähnlich der Technik von Garnspulen.

Figur 4 zeigt eine abgewandelte, zweite Ausführungsform eines Filtermoduls 110, wobei identische bzw. vergleichbare Merkmale mit identischen Bezugszeichen versehen sein. Die zweite Ausführungsform entspricht im Wesentlichen bis auf nachstehende Abweichungen der in Figur 1 gezeigten Ausführungsform.

Alternativ kann durch eine geeignete Kappe 60 das Blut durch ein zentrales Rohr 90, dass auch auf dem Umfang des Gehäuses 80 liegend angeordnet sein könnte, auf die Eintrittsseite zur Kappe 50 zurückgeführt werden. Der Vorteil einer solchen Konfiguration liegt darin, dass alle Anschlüsse 52, 54, 62 und 82 auf einer Seite des Moduls 110 sind.

Die Kappe 50 mit der Zuführung für Blut und Dialysat erhält einen zusätzlichen Dichtring 74, um den Blutauslass vom Dialysatraum zu trennen.

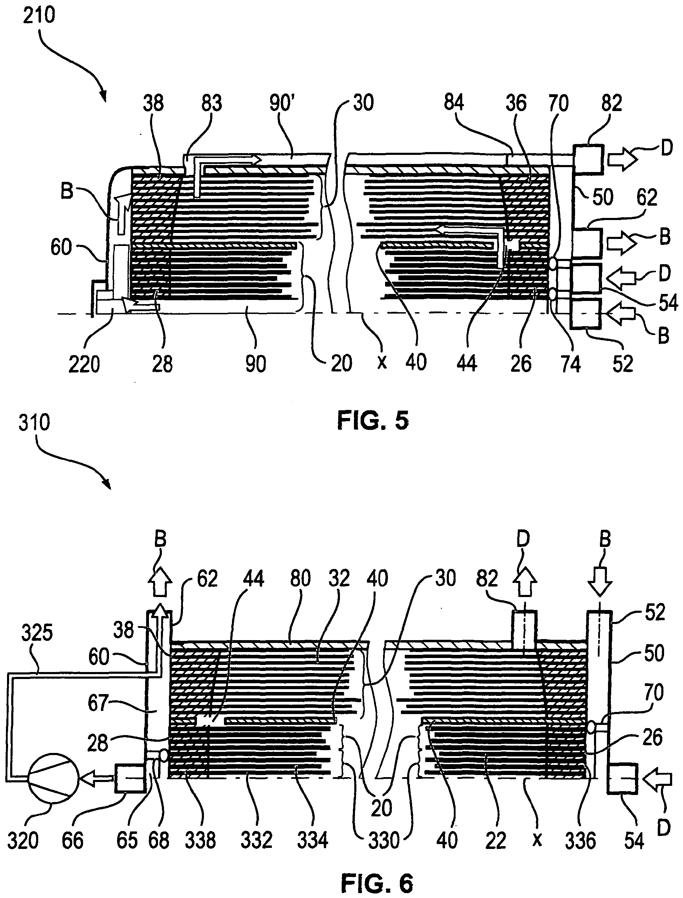

Figur 5 zeigt eine abgewandelte, dritte Ausführungsform eines Filtermoduls 210, wobei identische bzw. vergleichbare Merkmale mit identischen Bezugszeichen versehen sein. Auch die dritte Ausführungsform entspricht im Wesentlichen bis auf nachstehende Abweichungen der in Figur 1 und 2 gezeigten Ausführungsform. Der Blutzulauf in die zweite Filterstufe 30 erfolgt über den Kanal 90 und eine Umlenkung des Blutstroms B in der Kappe 60.

In die Kappe 60 kann zusätzlich der Rotor einer (Impeller)pumpe 220 integriert und der Fluss im Filtermodul 210 als mögliche, aber nicht notwendige Modifikation umgedreht werden. Um die Flussumkehr zu erreichen, werden die Öffnungsschlitze 44 für den internen Dialysataustritt Richtung Dialysateingang 54 verlagert, sowie der interne Dialysatausgang 83 Richtung Pumpenkappe 60 verschoben.

Um alle Anschlüsse auf einer Seite zu haben, wird bei dieser Variante das Dialysat auf dem Umfang des Gehäuses 80 in einem Kanal 90' zurückgeführt. Dieser Kanal 90' mündet in die Anschlusskappe 50 im Anschluss 82 bzw. dem Dialysatauslass 82.

Um die Entlüftung des Dialysatraums zu erleichtern, kann eine kleine Bohrung 84 zwischen außenliegendem Dialysatkanal 90' und innerem Dialysatraum kurz vor der Verbindungsstelle mit der Endkappe 50 vorgesehen werden. Die Bohrung 84 muss klein gehalten werden, um den daraus resultierenden Bypassstrom von Dialysat zu minimieren.

Figur 6 zeigt eine abgewandelte, dritte Ausführungsform eines Filtermoduls 310, wobei identische bzw. vergleichbare Merkmale mit identischen Bezugszeichen versehen sein. Der grundsätzliche Aufbau ist identisch zu dem in Figur 1 gezeigten Ausführungsbeispiel.

Der Kern des Hohlfasermembranbündels besteht in dieser Variante mit 2 Ultrafiltern gebildet durch die erste Filterstufe 20 und die dritte Filterstufe 330 dadurch, dass die innersten Fasern 332 der dritten Filterstufe 330 im Vergleich zu den sie umgebenden Fasern 22 der ersten Filterstufe 20 auf der einen Seite verschlossene Enden 336, bzw. auf der gegenüberliegenden Seite offene Enden 338 aufweisen. Die Gehäusekappe 60 auf der Blutaustrittsseite hat im zentralen Bereich eine zusätzliche Kammer 65, durch die Infusat zur Pumpe 320 und von dort über die Leitung 325 in den Blutbereich 67 fließen kann. Diese Kammer 65 ist mit einer Dichtung 68 vom Blutbereich 67 getrennt.

Ein Teilstrom des Dialysates umspült die Hohlfasermembranen 32 der zweiten Filterstufe 30 wie in der in Figur 1 gezeigten Ausführungsform und tritt über den sich am Umfang befindenden Konnektor 82 wieder aus. Ein zweiter Teilstrom an filtriertem Dialysat wird im Zentrum des Filtermoduls 310 durch die Wandung der Membranen 22 auf die Lumenseite der dritten Filterstufe gezogen. Die notwendige Druckdifferenz stellt eine externe (oder in die Kappe integrierte Pumpe ähnlich der Pumpe 220 gemäß dem in Figur 5 gezeigten Ausführungsbeispiel) her. Die Pumpe 320 stellt den Unterdruck im Bündels 334, also in der dritten Filterstufe 330 her, da diese Fasern 332 auf der Austrittsseite offene Enden 338 aufweisen.

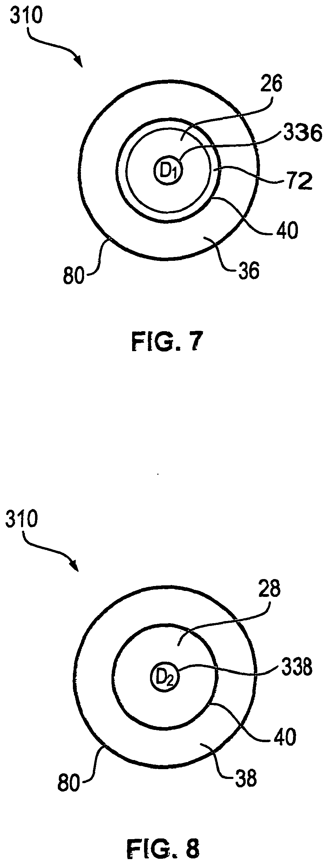

Der Austrittsdurchmesser D2 dieses Faserbündels ist kleiner (vgl. Figuren 7 und 8) als der Kerndurchmesser des vergossenen Bündels auf der Eintrittsseite D1. Damit wird gewährleistet, dass Dialysat immer in zwei Stufen gefiltert wird, bevor es z. B. als Post- und/oder Predilution infudiert wird. Es wird also der Austrittsdurchmesser D2 der dritten Filterstufe 330 kleiner ausgeführt als der Kerndurchmesser D1 der dritten Filterstufe 330 im Eintrittsbereich. Dadurch wird erreicht, dass einzelne Fasern, die durch die Produktion unversehens schräg in dem Gesamtbündel liegen, nicht von der dritten Filterstufe 330 in die erste Filterstufe 20 hineinragen. Dadurch wird vorteilhafterweise eine zweistufige Filtration gewährleistet.

## Patentansprüche

1. Filtermodul (10, 110, 210, 310) mit wenigstens einer ersten und wenigstens einer zweiten Filterstufe (20, 30), die im Wesentlichen konzentrisch angeordnet sind, wobei die Filterstufen (200, 30) Hohlfasermembranen (22, 32) umfassen und/oder aus diesen bestehen, wobei die Faserenden (26, 28, 36, 38) der Hohlfasermembranen (22, 32) zumindest teilweise und/oder zumindest einseitig in einer Vergussmasse eingebettet sind, wobei die erste Filterstufe (20) eine Filterstufe zur Filtration einer ersten medizinischen Flüssigkeit und die zweite Filterstufe (30) eine Filterstufe zur Filtration einer zweiten medizinischen Flüssigkeit ist, wobei die erste Filterstufe (20) einseitig verschlossene Faserenden (28) aufweist, wobei ein die erste Filterstufe (20) umgebendes Begrenzungsmittel (40) vorgesehen ist, das die erste Filterstufe (20) und die zweite Filterstufe (30) zumindest teilweise voneinander trennt und wenigstens eine Öffnung (44) aufweist, die eine Fluidverbindung zwischen erster Filterstufe (20) und zweiter Filterstufe (30) herstellt, wobei dieöffnung (44) im Begrenzungsmittel (40) aus einer oder einer Mehrzahl von umfänglich an dem Begrenzungsmittel (40) angeordneten Öffnungen (44) gebildet ist, wobei vorzugsweise die Öffnungen (44) umfänglich angeordnete Schlitze, Perforierungen, Löcher, Labyrinthschlitze oder dergleichen sind.

2. Filtermodul (10, 110, 210, 310) nach Anspruch 1, **dadurch gekennzeichnet, dass** das Begrenzungsmittel (40) wenigstens eine Entlüftungsöffnung (42) aufweist.

3. Filtermodul (10,110,210, 310) nach Anspruch 1 bis 2, **dadurch gekennzeichnet, dass** das Begrenzungsmittel (40) eine Begrenzungswand aufweist und dass die Öffnung (44) im Begrenzungsmittel (40) in der Begrenzungswand im zur Vergussmasse benachbarten Bereich angeordnet ist.

4. Filtermodul (10,110,210, 310) nach Anspruch 1 bis 3, **dadurch gekennzeichnet, dass** das Begrenzungsmittel (40) eine Folie und/oder ein Rohr ist oder umfasst.

5. Filtermodul (10, 110, 210, 310) nach einem der vorhergehenden Ansprüche, **dadurch gekennzeichnet, dass** das Filtermodul (10, 110, 210, 310) eine erste Endkappe (50) aufweist, in der ein Anschluss (52) zur ersten Filterstufe (20) und ein Anschluss (54) zur zweiten Filterstufe (30) angeordnet ist, wobei die Faserenden (26) der ersten Filterstufe (20) auf der Seite der ersten Endkappe (50) unverschlossen sind und wobei wenigstens ein Dichtelement (70) vorgesehen ist, mittels dessen die unverschlossenen Faserenden (26) der ersten Filterstufe (20) vom Anschluss zur zweiten Filterstufe (30) und von der zweiten Filterstufe (30) abdichtbar und/oder abgedichtet sind, wobei vorzugsweise das Filtermodul (10, 310) eine zweite Endkappe (60) aufweist, in der ein Anschluss (62) zur zweiten Filterstufe (30) angeordnet ist, und/oder die Faserenden (28) der ersten Filterstufe (20) auf der Seite der zweiten Endkappe (60) verschlossen sind.

6. Filtermodul (10, 110, 210, 310) nach einem der vorhergehenden Ansprüche, **dadurch gekennzeichnet, dass** das Filtermodul (10, 110, 210, 310) ein Gehäuse (80) aufweist, in dem die Filterstufen (20, 30, 330) angeordnet sind, wobei im Gehäuse (80) wenigstens ein Anschluss (82) zur ersten Filterstufe (20) angeordnet ist.

7. Filtermodul (110, 210) nach einem der vorhergehenden Ansprüche, **dadurch gekennzeichnet, dass** konzentrisch und koaxial zur Längsachse des Filtermoduls (110, 210) und/oder zu den Filterstufen (20, 30, 330) wenigstens ein Kanalelement (90) vorgesehen ist, das mit der zweiten Filterstufe (20) verbunden ist und/oder dass auf der Außenseite des Filtermoduls (110, 210) wenigstens ein Kanalelement vorgesehen ist, das mit der zweiten Filterstufe (30) verbunden ist.

8. Filtermodul (210, 310) nach einem der vorhergehenden Ansprüche, **dadurch gekennzeichnet, dass** das Filtermodul (210, 310) wenigstens ein Pumpenelement (220, 320) aufweist und/oder mit wenigstens einem Pumpenelement (220, 320) verbindbar ist, mittels dessen die erste und/oder zweite medizinische Flüssigkeit pumpbar ist, wobei vorzugsweise das Pumpenelement (220, 320) ein in das Filtermodul (210, 310) integriertes Pumpenelement (220, 320) ist und/oder das Pumpenelement (220, 320) eine Impellerpumpe ist.

9. Filtermodul (310) nach einem der vorhergehenden Ansprüche, **dadurch gekennzeichnet, dass** eine dritte Filterstufe (330) vorgesehen ist, wobei die erste, zweite und dritte Filterstufe (20, 30, 330) konzentrisch und koaxial angeordnet sind, wobei die erste Filterstufe (20) die dritte Filterstufe (330) umgibt und die zweite Filterstufe (30) die erste Filterstufe (20) umgibt.

10. Filtermodul (310) nach Anspruch 9, **dadurch gekennzeichnet, dass** das Filtermodul (310) eine erste Endkappe (50) aufweist, in der ein Anschluss (54) zur ersten Filterstufe (20) und ein Anschluss (52) zur zweiten Filterstufe (30) angeordnet ist, wobei die Faserenden (26) der ersten Filterstufe (20) auf der Seite der ersten Endkappe (50) unverschlossen sind und wobei wenigstens ein Dichtelement (70) vorgesehen ist, mittels dessen die unverschlossenen Faserenden (26) der ersten Filterstufe (20) vom Zulauf zur ersten Filterstufe (20) und von der zweiten Filterstufe (30) abdichtbar und/oder abgedichtet sind, wobei die Faserenden (336) der dritten Filterstufe (330) auf der Seite der ersten Endkappe (50) verschlossen sind, wobei eine zweite Endkappe (60) vorgesehen ist, in der ein Anschluss zur dritten Filterstufe (330) angeordnet ist und wobei die Faserenden (338) der dritten Filterstufe (330) auf der Seite der zweiten Endkappe (60) unverschlossen sind, wobei vorzugsweise an den Anschluss (60) zur dritten Filterstufe (330) wenigstens ein Pumpenelement (320) anschließbar und/oder angeschlossen ist, wobei das Pumpenelement (320) vorzugsweise ein integriertes Pumpenelement (320) ist, und/oder wobei der Anschluss (66) zur dritten Filterstufe (330) mit einem Anschluss (62) zur zweiten Filterstufe (30) verbindbar und/oder verbunden ist.

11. Verfahren zur Herstellung eines Hohlfasermembranbündels zur Anwendung in dem Filtermodul der Ansprüche 1 bis 10 mit wenigstens zwei konzentrisch angeordneten Filterstufen (20, 30), das wenigstens die folgende Schritte umfasst:
- Einfassen eines ersten Hohlfasermembranbündels für eine erste Filterstufe in eine Folie, Schlauchfolie und/oder ein Rohr;
- Einbetten der ersten Filtrationsstufe (20) in Hohlfasern der zweiten Filtrationsstufe (30);
- Vergießen der ersten und der zweiten Filtrationsstufe (20, 30) durch eine Vergussmasse in einem Gießprozess;
- Abschneiden eines Endbereichs der Vergussmasse inklusive verschlossener Faserenden zum Freilegen und Offenlegen aller Faserenden; und
- Verschließen einer Faserendenseite der ersten Filtrationsstufe (20) durch Wärmeeinwirkung und Schmelzen der Faserendenseite und Vergussmasse.

12. Verfahren nach Anspruch 11, **dadurch gekennzeichnet, dass** das Verschließen der Faserendenseite mittels Laser und/oder Heißstempel erfolgt.

13. Verfahren nach Anspruch 11 oder 12, **dadurch gekennzeichnet, dass** in der ersten Filtrationsstufe (20) eine dritte Filtrationsstufe (330) ausgebildet wird, wobei auf einer Faserendenseite ein erster Bereich (D1) verschlossen wird und wobei auf der anderen Faserendenseite ein zweiter Bereich verschlossen wird, wobei im ersten Bereich (D1) Hohlfasern verschlossen werden und diese Hohlfasern auf der anderen Faserendenseite vom zweiten Bereich umgeben und zumindest teilweise unverschlossen sind, wobei vorzugsweise der erste Bereich (D1) kreisförmig und dass der zweite Bereich kreisringförmig ist, wobei vorzugsweise der Innendurchmesser des zweiten Bereichs kleiner gewählt ist als der Außendurchmesser des ersten Bereichs (D1).

14. Verfahren nach einem der Ansprüche 11 bis 13, **dadurch gekennzeichnet**, in wenigstens einem weiteren Schritt aus dem Hohlfasermembranbündel ein Filtermodul (10, 110, 210, 310) nach einem der Ansprüche 1 bis 10 hergestellt wird.

15. Disposable, umfassend wenigstens ein Filtermodul (10, 110, 210, 310) nach einem der Ansprüche 1 bis 10, wobei das Disposable eine disposable Kassette ist und/oder umfasst.

16. Dialysemaschine mit wenigstens einem Filtermodul (10, 110, 210, 310) nach einem der Ansprüche 1 bis 10 und/oder wenigstens einem Disposable nach Anspruch 15.

## Claims

1. A filter module (10, 110, 210, 310) having at least one first and at least one second filter stage (20, 30) which are substantially concentrically arranged, the filter stages (200, 30) comprising and/or consisting of hollow fiber membranes (22, 32), wherein the fiber ends (26, 28, 36, 38) of the hollow fiber membranes (22, 32) are embedded at least partly and/or at least unilaterally in a casting compound, wherein the first filter stage (20) is a filter stage for the filtration of a first medical fluid and the second filter stage (30) is a filter stage for the filtration of a second medical fluid, wherein the first filter stage (20) has unilaterally closed fiber ends (28), wherein a boundary means (40) is provided which surrounds the first filter stage (20) and which separates the first filter stage (20) and the second filter stage (30) at least partly from one another and has at least one opening (44) which establishes a fluid communication between the first filter stage (20) and the second filter stage (30), wherein the opening (44) in the boundary means (40) is formed from one or from a plurality of openings (44) arranged peripherally at the boundary means (40), with the openings (44) preferably being peripherally arranged slits, perforations, holes, labyrinth slits or the like.

2. A filter module (10, 110, 210, 310) in accordance with claim 1, **characterized in that** the boundary means (40) has at least one venting opening (42).

3. A filter module (10, 110, 210, 310) in accordance with claims 1 to 2, **characterized in that** the boundary means (40) has a boundary wall; and **in that** the opening (44) in the boundary means (40) in the boundary wall is arranged in the region adjacent to the casting compound.

4. A filter module (10, 110, 210, 310) in accordance with claims 1 to 3, **characterized in that** the boundary means (40) is or includes a film and/or a tube.

5. A filter module (10, 110, 210, 310) in accordance with one of the preceding claims, **characterized in that** the filter module (10, 110, 210, 310) has a first end cap (50) in which a connection (52) to the first filter stage (20) and a connection (54) to the second filter stage (300) is arranged, with the fiber ends (26) of the first filter stage (20) being unclosed on the side of the first end cap (50) and with at least one sealing element (70) being provided by means of which the unclosed fiber ends (26) of the first filter stage (20) can be and/or are sealed from the connection to the second filter stage (30) and from the second filter stage (30), with the filter module (10, 310) preferably having a second end cap (60) in which a connection (62) to the second filter stage (30) is arranged; and/or with the fiber ends (28) of the second filter stage (20) being closed on the side of the second end cap (60).

6. A filter module (10, 110, 210, 310) in accordance with one of the preceding claims, **characterized in that** the filter module (10, 110, 210, 310) has a housing (80) in which the filter stages (20, 30, 330) are arranged, with at least one connection (82) to the first filter stage (20) being arranged in the housing (80).

7. A filter module (110, 210) in accordance with one of the preceding claims, **characterized in that** at least one passage element (90) is provided concentrically and coaxially to the longitudinal axis of the filter module (110, 210) and/or to the filter stages (20, 30, 330) and is connected to the second filter stage (20); and/or **in that** at least one passage element which is connected to the second filter stage (30) is provided on the outer side of the filter module (110, 210).

8. A filter module (210, 310) in accordance with one of the preceding claims, **characterized in that** the filter module (210, 310) has at least one pump element (220, 320) and/or can be connected to at least one pump element (220, 320) by means of which the first medical fluid and/or the second medical fluid can be pumped, with the pump element (220, 320) preferably being a pump element (220, 320) integrated into the filter module (210, 310); and/or with the pump element (220, 320) being an impeller pump.

9. A filter module (310) in accordance with one of the preceding claims, **characterized in that** a third filter stage (330) is provided, with the first, second and third filter stages (20, 30, 330) being arranged concentrically and coaxially, with the first filter stage (20) surrounding the third filter stage (330) and the second filter stage (30) surrounding the first filter stage (20).

10. A filter module (310) in accordance with claim 9, **characterized in that** the filter module (310) has a first end cap (50) in which a connection (54) to the first filter stage (20) and a connection (52) to the second filter stage (30) is arranged, with the fiber ends (26) of the first filter stage (20) being unclosed on the side of the first end cap (50) and with at least one sealing element (70) being provided by means of which the unclosed fiber ends (26) of the first filter stage (20) can be and/or are sealed from the inflow to the first filter stage (20) and from the second filter stage (30), with the fiber ends (336) of the third filter stage (330) being closed on the side of the first end cap (50), with a second end cap (60) being provided in which a connection to the third filter stage (330) is arranged and with the fiber ends (338) of the third filter stage (330) being unclosed on the side of the second end cap (60), with at least one pump element (320) preferably being connectable and/or connected to the connection (60) to the third filter stage (330), with the pump element (320) preferably being an integrated pump element (320) and/or with the connection (66) to the third filter stage (330) being connectable and/or connected to a connection (62) to the second filter stage (30).

11. A method of manufacturing a hollow fiber membrane bundle for use in the filter module in accordance with claims 1 to 10 having at least two concentrically arranged filter stages (20, 30) which includes at least the following steps:
- surrounding a first hollow fiber membrane bundle for a first filter stage in a film, tube film and/or a tube;
- embedding the first filtration stage (20) in hollow fibers of the second filtration stage (30);
- molding the first and second filtration stages (20, 30) by a casting compound in a molding process;
- cutting off an end region of the casting compound, including closed fiber ends, for exposing and laying open all fiber ends; and
- closing a fiber end side of the first filtration stage (20) by thermal influence and melting the fiber end side and the casting compound.

12. A method in accordance with claim 11, **characterized in that** the closing of the fiber end side takes place by means of laser and/or hot stamp.

13. A method in accordance with either of claims 11 or 12, **characterized in that** a third filtration stage (330) is formed in the first filtration stage (20), with a first region (D1) being closed on one fiber end side and with a second region being closed on the other fiber end side and with hollow fibers being closed in the first region (D1) and these hollow fibers being surrounded by the second region and being at least partly unclosed on the other fiber end side, with the first region (D1) preferably being circular, and with the second region preferably being annular, with the inner diameter of the second region preferably being selected to be smaller than the outer diameter of the first region (D1).

14. A method in accordance with one of the claims 11 to 13, **characterized in that** a filter module (10, 110, 210, 310) in accordance with one of the claims 1 to 10 is manufactured from the hollow fiber membrane bundle in at least one further step.

15. A disposable comprising at least one filter module (10, 110, 210, 310) in accordance with one of the claims 1 to 10, wherein the disposable is and/or comprises a disposable cassette.

16. A dialysis machine having at least one filter module (10, 110, 210, 310) in accordance with one of the claims 1 to 10 and/or having at least one disposable in accordance with claim 15.

## Revendications

1. Module de filtrage (10, 110, 210, 310) comprenant au moins un premier et au moins un deuxième étage de filtrage (20, 30), qui sont disposés de manière sensiblement concentrique, les étages de filtrage (200, 30) comprenant des membranes à fibres creuses (22, 32) et/ou étant constitués de celles-ci, les extrémités de fibres (26, 28, 36, 38) des membranes à fibres creuses (22, 32) étant incorporées au moins en partie et/ou au moins d'un côté dans une masse de remplissage, le premier étage de filtrage (20) étant un étage de filtrage destiné au filtrage d'un premier liquide médical et le deuxième étage de filtrage (30) étant un étage de filtrage destiné au filtrage d'un second liquide médical, le premier étage de filtrage (20) comportant des extrémités de fibres (28) obturées d'un côté, un moyen de limitation (40) entourant le premier étage de filtrage (20) étant prévu, qui sépare le premier étage de filtrage (20) et le deuxième étage de filtrage (30) au moins en partie l'un de l'autre et comporte au moins un orifice (44), qui établit une liaison fluidique entre le premier étage de filtrage (20) et le deuxième étage de filtrage (30), l'orifice (44) dans le moyen de limitation (40) étant formé par un ou par plusieurs orifices (44) disposés sur la circonférence du moyen de limitation (40), les orifices (44) étant de préférence des fentes, des perforations, des trous, des fentes en labyrinthe ou similaires disposés sur la circonférence.

2. Module de filtrage (10, 110, 210, 310) selon la revendication 1, **caractérisé en ce que** le moyen de limitation (40) comporte au moins un orifice d'évacuation d'air (42).

3. Module de filtrage (10, 110, 210, 310) selon les revendications 1 à 2, **caractérisé en ce que** le moyen de limitation (40) comporte une paroi de limitation et **en ce que** l'orifice (44) dans le moyen de limitation (40) est disposé dans la paroi de limitation dans la zone avoisinant la masse de remplissage.

4. Module de filtrage (10, 110, 210, 310) selon les revendications 1 à 3, **caractérisé en ce que** le moyen de limitation (40) est ou comprend une feuille et/ou un tube.

5. Module de filtrage (10, 110, 210, 310) selon l'une des revendications précédentes, **caractérisé en ce que** le module de filtrage (10, 110, 210, 310) comporte un premier embout (50), dans lequel est disposé un raccordement (52) avec le premier étage de filtrage (20) et un raccordement (54) avec le deuxième étage de filtrage (30), les extrémités de fibres (26) du premier étage de filtrage (20) n'étant pas obturées du côté du premier embout (50) et au moins un élément d'étanchéité (70) étant prévu, au moyen duquel les extrémités de fibres (26) non obturées du premier étage de filtrage (20) peuvent être et/ou sont rendues étanches par rapport au raccordement avec le deuxième étage de filtrage (30) et au deuxième étage de filtrage (30), le module de filtrage (10, 310) comportant de préférence un second embout (60), dans lequel est disposé un raccordement (62) avec le deuxième étage de filtrage (30), et/ou les extrémités de fibres (28) du premier étage de filtrage (20) étant obturées du côté du second embout (60).

6. Module de filtrage (10, 110, 210, 310) selon l'une des revendications précédentes, **caractérisé en ce que** le module de filtrage (10, 110, 210, 310) comporte un boîtier (80), dans lequel les étages de filtrage (20, 30, 330) sont disposés, au moins un raccordement (82) avec le premier étage de filtrage (20) étant disposé dans le boîtier (80).

7. Module de filtrage (110, 210) selon l'une des revendications précédentes, **caractérisé en ce qu'**au moins un élément de conduit (90) est prévu de manière concentrique et coaxiale par rapport à l'axe longitudinal du module de filtrage (110, 210) et/ou par rapport aux étages de filtrage (20, 30, 330), ledit élément de conduit étant relié au deuxième étage de filtrage (20) et/ou **en ce qu'**au moins un élément de conduit est prévu sur le côté extérieur du module de filtrage (110, 210), ledit élément de conduit étant relié au deuxième étage de filtrage (30).

8. Module de filtrage (210, 310) selon l'une des revendications précédentes, **caractérisé en ce que** le module de filtrage (210, 310) comporte au moins un élément de pompe (220, 320) et/ou peut être relié à au moins un élément de pompe (220, 320), au moyen duquel le premier et/ou le second liquide médical peuvent être pompés, l'élément de pompe (220, 320) étant de préférence un élément de pompe (220, 320) intégré dans le module de filtrage (210, 310) et/ou l'élément de pompe (220, 320) étant une pompe centrifuge.

9. Module de filtrage (310) selon l'une des revendications précédentes, **caractérisé en ce qu'**un troisième étage de filtrage (330) est prévu, le premier, deuxième et troisième étage de filtrage (20, 30, 330) étant disposés de manière concentrique et coaxiale, le premier étage de filtrage (20) entourant le troisième étage de filtrage (330) et le deuxième étage de filtrage (30) entourant le premier étage de filtrage (20).

10. Module de filtrage (310) selon la revendication 9, **caractérisé en ce que** le module de filtrage (310) comporte un premier embout (50), dans lequel est disposé un raccordement (54) avec le premier étage de filtrage (20) et un raccordement (52) avec le deuxième étage de filtrage (30), les extrémités de fibres (26) du premier étage de filtrage (20) n'étant pas obturées du côté du premier embout (50) et au moins un élément d'étanchéité (70) étant prévu, au moyen duquel les extrémités de fibres (26) non obturées du premier étage de filtrage (20) peuvent être et/ou sont rendues étanches par rapport à l'arrivée dans le premier étage de filtrage (20) et au deuxième étage de filtrage (30), les extrémités de fibres (336) du troisième étage de filtrage (330) étant obturées du côté du premier embout (50), un second embout (60) étant prévu, dans lequel est disposé un raccordement avec le troisième étage de filtrage (330) et les extrémités de fibres (338) du troisième étage de filtrage (330) n'étant pas obturées du côté du second embout (60), au moins un élément de pompe (320) pouvant être et/ou étant de préférence relié au raccordement (60) avec le troisième étage de filtrage (330), l'élément de pompe (320) étant de préférence un élément de pompe (320) intégré, et/ou le raccordement (66) avec le troisième étage de filtrage (330) pouvant être et/ou étant relié au raccordement (62) avec le deuxième étage de filtrage (30).

11. Procédé de fabrication d'un groupe de membranes à fibres creuses destiné à être utilisé dans le module de filtrage selon les revendications 1 à 10, comprenant au moins deux étages de filtrage (20, 30) disposés de manière concentrique, comprenant au moins les étapes suivantes consistant à :
- ceindre un premier groupe de membranes à fibres creuses pour un premier étage de filtrage dans une feuille, une feuille en gaine et/ou un tube ;
- incorporer le premier étage de filtrage (20) dans des fibres creuses du deuxième étage de filtrage (30) ;
- enrober le premier et le deuxième étage de filtrage (20, 30) dans une masse de remplissage par un processus de coulage ;
- couper une zone d'extrémité de la masse de remplissage y compris les extrémités de fibres obturées pour dégager et découvrir toutes les extrémités de fibres ; et
- obturer un côté d'extrémités de fibres du premier étage de filtrage (20) par action de la chaleur et fusion du côté d'extrémités de fibres et de la masse de remplissage.

12. Procédé selon la revendication 11, **caractérisé en ce que** l'obturation du côté d'extrémités de fibres est effectuée au moyen d'un laser et/ou d'un poinçon chaud.

13. Procédé selon la revendication 11 ou 12, **caractérisé en ce qu'**un troisième étage de filtrage (330) est formé dans le premier étage de filtrage (20), une première zone (D1) étant obturée sur un côté d'extrémités de fibres et une seconde zone étant obturée sur l'autre côté d'extrémités de fibres, des fibres creuses étant obturées dans la première zone (D1) et ces fibres creuses étant entourées et au moins en partie non obturées par la seconde zone de l'autre côté d'extrémités de fibres, la première zone (D1) étant de préférence circulaire et **en ce que** la seconde zone est en forme d'anneau circulaire, le diamètre intérieur de la seconde zone étant de préférence sélectionné inférieur au diamètre extérieur de la première zone (D1).

14. Procédé selon l'une des revendications 11 à 13, **caractérisé en ce que**, dans au moins une autre étape, un module de filtrage (10, 110, 210, 310) selon l'une des revendications 1 à 10 est fabriqué à partir du groupe de membranes à fibres creuses.

15. Objet à emploi unique, comprenant au moins un module de filtrage (10, 110, 210, 310) selon l'une des revendications 1 à 10, dans lequel l'objet à emploi unique est et/ou comprend une cassette à emploi unique.

16. Machine de dialyse comprenant au moins un module de filtrage (10, 110, 210, 310) selon l'une des revendications 1 à 10 et/ou au moins un objet à emploi unique selon la revendication 15.
